Europäisches Patentamt

European Patent Office

Office européen des brevets

(11) Publication number: **0 102 012**
**B1**

# (12) EUROPEAN PATENT SPECIFICATION

(45) Date of publication of patent specification: **04.11.87**   (51) Int. Cl.⁴: **A 61 M 5/14**

(21) Application number: **83107980.1**

(22) Date of filing: **12.08.83**

(54) **Inflatable bag-presser unit for infusion bags.**

(30) Priority: **27.08.82 IL 66665**

(43) Date of publication of application:
**07.03.84 Bulletin 84/10**

(45) Publication of the grant of the patent:
**04.11.87 Bulletin 87/45**

(84) Designated Contracting States:
**AT BE CH DE FR GB IT LI NL SE**

(56) References cited:
**DE-A-2 006 054**
**DE-A-2 731 448**
**FR-A-1 346 117**
**FR-A-2 191 914**
**GB-A-2 069 841**
**LU-A- 68 188**
**US-A-2 766 907**
**US-A-4 090 514**

(73) Proprietor: **Leibinsohn, Saul**
**11 Olei Hagardom Street**
**Rishon Lezion (IL)**

(72) Inventor: **Leibinsohn, Saul**
**11 Olei Hagardom Street**
**Rishon Lezion (IL)**

(74) Representative: **Modiano, Guido et al**
**MODIANO, JOSIF, PISANTY & STAUB Modiano**
**& Associati Via Meravigli, 16**
**I-20123 Milan (IT)**

Courier Press, Leamington Spa, England.

## Description

The present invention relates to an inflatable bag-presser unit for inducing the flow of a liquid from an infusion bag. The invention is particularly applicable for use in the apparatus described in my UK Patent Application 8310283 of April 15, 1983, now UK Patent GB 2118634B published October 2, 1985, and is therefore described below with respect to this application, but it will be appreciated that the invention could be used in other applications as well.

The above-cited patent application describes apparatus for the induced infusion of a liquid from an infusion bag comprising a hand-operable pumping bulb, elastic inflatable means coupled to the bulb for inflation by a fluid pumped therein, and a sleeve for retaining the bag firmly pressed against a first portion of the elastic inflatable means for applying the pressure thereof to the bag in order to induce the flow of the liquid therefrom. As described in that application, a second portion of the inflatable means serves as an accumulator for accumulating pressurized fluid pumped by the bulb. An outer non-elastic sheath encloses the latter portion of the inflatable means and limits the accumulation of the pressurized fluid therein.

In the preferred embodiment of the invention described in that application, the first and second portions of the elastic inflatable means are separate inflatable units. Thus, the first portion is an inflatable bag-presser unit against which the liquid bag is pressed by the flexible sleeve, and the second portion is a separate inflatable accumulator unit enclosed by the mentioned outer sheath.

The present invention relates to a construction of an inflatable bag-presser unit particularly useful in the apparatus of that patent application.

Inflatable bag-presser units for use with an infusion bag are known in which the bag-presser unit comprises an inflatable bladder and a flexible sleeve adapted to receive the infusion bag and to retain it pressed against one side of the inflatable bladder, with the sleeve being in the form of a flexible strip wrappable around the inflatable bladder and the infusion bag; see for example USA Patent 2,766,907. The present invention provides an improvement in inflatable bag-presser units of that type.

However, prior bag pressure units for the induced infusion of a liquid from an infusion bag are not devoid of drawbacks, among which is the fact that, in a known manner, an inflatable bladder located in a bag presser unit, must be frequently monitored or re-pumped so as to maintain substantially constant pressure on the infusion bag, in order to achieve complete administration of its contents.

Another disadvantage of prior bag presser units is that they frequently imply danger of over-pressurizing the infusion liquid, particularly at the time of its administration.

A not least disadvantage of the prior bag pressure units is that they are often of large overall size.

Accordingly, it is an aim of the present invention to provide a bag presser unit which does not require constant monitoring to maintain substantially constant pressure on the infusion bag for the complete administration of its contents.

Within the above aim it is an object of the invention to provide a bag presser unit which lessens the danger of over-pressurizing the infusion liquid.

Another object of the invention is to provide a bag presser unit of reduced size.

This aim and these and other objects which will become apparent hereinafter are achieved by an inflatable bag-presser unit (204) for use with an infusion bag (202) comprising an inflatable bladder (206) and a sleeve (208) in the form of a flexible strip adapted to receive said infusion bag (202) and to retain it pressed against one side of said inflatable bladder (206), characterized in that said bag-presser unit further comprises a rigid backing plate (240) on the other side of said bladder, said sleeve (208) being secured at one end to one of said rigid backing plate (240) and being wrappable around said inflatable bladder (206), said infusion bag (202) and said rigid backing plate (240), and the other end of said sleeve (208) being attached by releasable fastening means (250, 252, 254, 256) to the side of the rigid backing plate (240) away from said inflatable bladder (206).

Although it is known, for example as shown in LU—A—68188, to use a rigid supporting member for an inflatable bag-presser unit, the foregoing arrangement not previously known, using a rigid backing plate and a flexible strip which is wrappable about the rigid backing plate, has been found to be very advantageous particularly when used in the apparatus of the above-cited British patent application.

In the preferred embodiment of the invention described herein, the rigid backing plate is bent to partially enclose the bladder and the infusion bag; also the rigid backing plate is enclosed within a flexible sleeve, the flexible strip being secured at one end to the rigid backing plate sleeve, and at the opposite end being attached to the rigid backing plate sleeve by releasable fastening means.

Further, in the described preferred embodiment, the inflatable bladder is also enclosed within a flexible sleeve and includes releasable fasteners for releasably attaching same to the rigid backing sleeve.

Further features of the invention will be apparent from the description below.

The invention is herein described, by way of example only, with reference to the accompanying drawings, wherein:

Fig. 1 illustrates one form of inflatable bag-presser unit constructed in accordance with the invention and particularly useful in the apparatus of the above-cited patent application; and

Fig. 2 is a transverse sectional view of the unit of Fig. 1 during its use for inducing flow from an infusion bag.

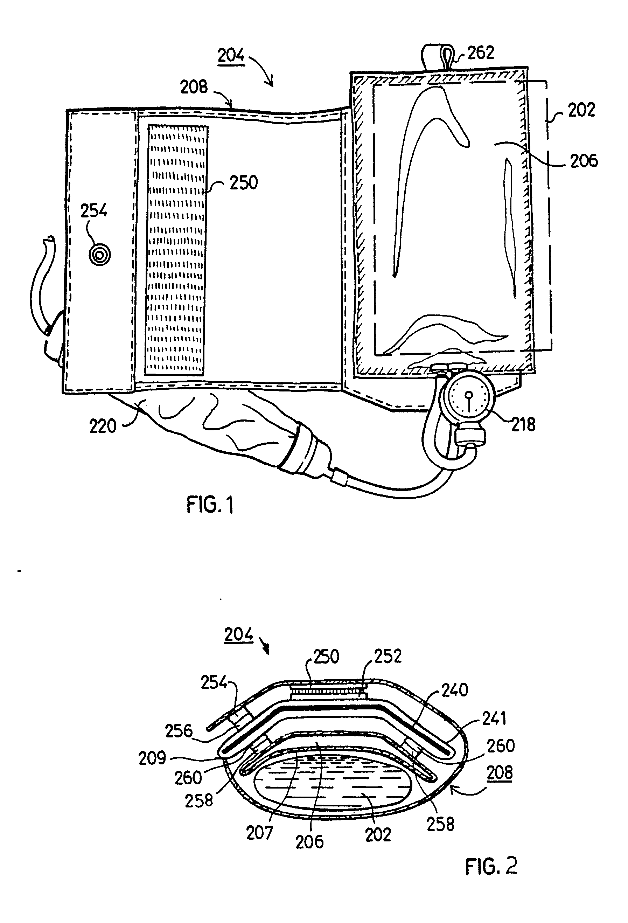

The bag-presser unit illustrated in Figs. 1 and 2 of the drawings is generally designated 204 and includes an inflatable bladder 206, a rigid backing plate 240 on one side of the bladder, and a flexible sleeve, generally designated 208, adapted to enclose the infusion bag 202 and to retain it pressed against the opposite side of the inflatable bladder 206. The bag-presser unit also includes an accumulator unit 220 which provides a number of important advantages: Thus, the accumulator 220 maintains the pressure on the infusion bag 202 substantially constant for the complete administration of the contents of the infusion bag, without the need to monitor or re-pump the inflatable bladder 206 in the bag-presser unit 204. Another advantage is that it lessens the danger of over-pressurizing the infusion liquid at the time of its administration; and a still further advantage is that it reduces the size of the bag-presser unit 204.

As shown in Fig. 2, the rigid backing plate 240 is bent at its opposite ends in order to partially enclose the inflatable bladder 206 and the infusion bag 202. The inflatable bladder 206 is enclosed within a flexible fabric sleeve 207; and similarly, the rigid backing plate 240 is also enclosed within a flexible fabric sleeve 241. Further, the overall sleeve 208 which encloses all the foregoing elements, including the infusion bag 202, is in the form of a flexible fabric strip secured at one end 209 to one end of the flexible sleeve 241 of the backing plate 240. Flexible strip 208 is then wrapped around the opposite end of the rigid backing plate 240 and is releasably attached by releasable fastening means to the upper face of the rigid backing plate sleeve 241.

As also shown in Fig. 2, the releasable fastening means comprises a plastic strip 250 of interlocking hooks and projections secured to the inner face of the flexible strip 208, and a mating plastic strip 252 secured to the outer face of the rigid backing plate sleeve 241. These interlocking plastic strips may be "Velcro" (Reg. T.M.) strips.

The releasable fastening means further includes snap-fasteners 254 and 256 applied to the outer extremity of the outer strip 208 and the outer face of the rigid backing plate sleeve 241. In addition, sleeve 207 for the inflatable bladder 206 is also provided with releasable snap-fasteners 258 cooperable with fasteners 260 on the inner face of the rigid backing plate sleeve 241, to permit attachment and detachment of the inflatable bladder to the rigid backing plate. Further, the flexible sleeve 207 for the bladder 206 includes a loop 262 (Fig. 1) for suspending the inflatable bag-presser unit during use.

In order to use the illustrated device, inflatable bladder 206 is first attached to sleeve 241 of the rigid backing plate 240 by fasteners 258 and 260; the infusion bag 202 is placed against the inflatable bladder 206; and the flexible strip 208 is then wrapped around the infusion bag 202 and is secured to the unit in the form of a sleeve by the plastic strip fasteners 250, 252, and by the snap-fastener 254, 256.

## Claims

1. An inflatable bag-presser unit for use with an infusion bag, comprising an inflatable bladder and a sleeve in the form of a flexible strip adapted to receive said infusion bag and to retain it pressed against one side of said inflatable bladder; characterized in that said bag-presser unit further comprises a rigid backing plate on the other side of said bladder, said sleeve being secured at one end to one end of said rigid backing plate and being wrappable around said inflatable bladder, said infusion bag and said rigid backing plate, and the other end of said sleeve being attached by releasable fastening means to the side of the rigid backing plate away from said inflatable bladder.

2. The bag-presser unit according to Claim 1, wherein the opposite ends of said rigid backing plate (240) are bent to partially enclose said bladder (206) and said infusion bag (202).

3. The bag-presser unit according to either of Claims 1 or 2, wherein said rigid backing plate (240) is enclosed within a flexible sleeve (241), said flexible strip (208) being secured at one end (209) to said backing plate sleeve (241), and at the opposite end being attached to the backing plate sleeve (241) by releasable fastening means (250, 252, 254, 256).

4. The bag-presser unit according to Claim 3, wherein said releasable fastening means comprises snap-fasteners (254, 256) between the inner face of the flexible strip (208) and the outer face of the backing plate sleeve (241).

5. The bag-presser unit according to either of Claims 3 and 4, wherein said releasable fastening means comprises strips (250, 252) of interlocking plastic hooks and projections on the inner face of said flexible strip (208) and on the outer face of said backing plate sleeve (241).

6. The bag-presser unit according to any one of Claims 3—5, wherein said inflatable bladder (206) is also enclosed within a flexible sleeve (207) and includes releasable fasteners (258, 260) for releasably attaching said bladder (206) to the backing plate sleeve (241).

7. The bag-presser unit according to Claim 6, wherein said flexible sleeve (207) includes a loop (262) for suspending the inflatable bag-presser unit (204) and the infusion bag (202) during use.

## Patentansprüche

1. Aufblasbare Vorrichtung zum Ausüben von Druck auf Infusionsbeutel, bestehend aus einer aufblasbaren Blase und einer Manschette in der Form eines biegsamen Bandes, das befähigt ist, den Infusionsbeutel aufzunehmen und gegen eine Seite der aufblasbaren Blase gedrückt zu halten, dadurch gekennzeichnet, daß die Vorrichtung zur Druckausübung weiters eine starre Stützplatte an der anderen Seite der Blase aufweist und die Manschette mit einem Ende an einem Ende der starren Stützplatte befestigt und um die aufblasbare Blase wickelbar ist, wobei der Infusionsbeutel, die starre Stützplatte und das andere Ende der Manschette durch lösbaren Befestigungseinrichtungen mit der zur aufblasbaren Blase abgelegenen Seite der starren Stützplatte verbunden sind.

2. Vorrichtung nach Anspruch 1, dadurch gekennzeichnet, daß die entgegengesetzten Enden der starren Stützplatte (240) abgewinkelt sind, um die Blase (206) und den Infusionsbeutel (202) teilweise zu umfassen.

3. Vorrichtung nach Anspruch 1 oder 2, dadurch gekennzeichnet, daß die starre Stützplatte (240) in einer biegsamen Hülle (241) eingeschlossen ist, wobei das biegsame Band (208) mit einem Ende (209) an der Stützplattenhülle (241) befestigt und an seinem entgegengesetzte Ende durch lösbare Befestigungseinrichtungen (250, 252, 254, 256) mit der Stützplattenhülle (241) verbunden ist.

4. Vorrichtung nach Anspruch 3, dadurch gekennzeichnet, daß die lösbaren Befestigungseinrichtungen aus Schnappverschlüssen (254, 256) zwischen der Innenfläche des biegsamen Bandes (208) und der Außenfläche der Stützplattenhülle (241) bestehen.

5. Vorrichtung nach einem der Ansprüche 3 und 4, dadurch gekennzeichnet, daß die lösbaren Befestigungseinrichtungen aus Klettenverschlußstreifen (250, 252) auf der Innenfläche des biegsamen Bandes (208) und auf der Außenfläche der Stützplattenhülle (241) bestehen.

6. Vorrichtung nach einem der Ansprüche 3—5, dadurch gekennzeichnet, daß die aufblasbare Blase (206) ebenfalls in einer biegsamen Hülle (207) eingeschlossen ist und lösbare Befestigungseinrichtungen (258; 260) zur lösbaren Verbindung der Blase (206) mit der Stützplattenhülle (241) aufweist.

7. Vorrichtung nach Anspruch 6, dadurch gekennzeichnet, daß die biegsame Hülle (207) eine Schlaufe (262) zum Aufhängen der Vorrichtung zur Druckausübung (204) und des Infusionsbeutels (202) während der Benützung aufweist.

**Revendications**

1. Elément presseur pour poche gonflable prévu pour être utilisé avec une poche de perfusion, comportant une vessie gonflable et un manchon sous forme d'une bande souple prévue pour recevoir la poche de perfusion et pour la maintenir pressée contre une face de la vessie gonflable, caractérisé en ce qu'il comporte en outre une plaque de renforcement rigide sur l'autre face de la vessie gonflable, le manchon étant fixé à une extrémité de la plaque de renforcement rigide et pouvant envelopper la vessie gonflable, la poche de perfusion et la plaque de renforcement rigide, et l'autre extrémité du manchon étant reliée, à l'aide de moyens de fixation séparables, à la face de la plaque de renforcement rigide éloignée de la vessie gonflable.

2. Elément presseur pour poche gonflable selon la revendication 1, dans lequel les extrémités opposées de la plaque de renforcement rigide (240) sont cintrées de façon à envelopper partiellement la vessie (206) et la poche de perfusion (202).

3. Elément presseur pour poche gonflable selon l'une des revendications 1 ou 2 dans lequel la plaque de renforcement rigide (240) est enveloppée dans un manchon souple (241), la bande souple (208) étant fixée à une extrémité (209) du manchon (241) de la plaque de renforcement, et étant reliée à l'extrémité opposée au manchon (241) de la plaque de renforcement par des moyens de fixation séparables (250, 252, 254, 256).

4. Elément presseur pour poche gonflable selon la revendication 3 dans lequel les moyens de fixation séparables comportent des boutons-pression (254, 256) entre la face intérieure de la bande souple (208) et la face extérieure du manchon (241) de la plaque de renforcement.

5. Elément presseur pour poche gonflable selon l'une des revendications 3 et 4 dans lequel les moyens de fixation séparables comprennent des bandes (250, 252) de crochets plastiques d'interconnexion et des saillies sur la face intérieure de la bande souple (208) et sur la face extérieure du manchon (241) de la plaque de renforcement.

6. Elément presseur pour poche gonflable selon l'une quelconque des revendications 3 à 5 dans lequel la vessie gonflable (206) est également enveloppée à l'intérieur d'un manchon souple (207) et comporte des moyens de fixation séparables (258, 260), de façon à relier de façon séparable la vessie (206) au manchon (241) de la plaque de renforcement.

7. Elément presseur pour poche gonflable selon la revendication 6 dans lequel le manchon souple (207) comporte une boucle (262) permettant de suspendre, pendant l'emploi, l'élément presseur pour poche gonflable (204) et la poche de perfusion (202).

FIG. 1

FIG. 2